# EUROPEAN PATENT APPLICATION

(11) **EP 3 275 502 A1**
(43) Date of publication of application: **31.01.2018**
(21) Application number: 17183556.4
(22) Date of filing: 27.07.2017
(51) Int. Cl.: A61M 37/00

(54) **METHOD OF PRODUCING TRANSDERMAL ABSORPTION SHEET**

(30) Priority: 28.07.2016 JP 2016148779
(71) Applicant: Fujifilm Corporation, Minato-ku Tokyo 106-8620 (JP)
(72) Inventor: URABE, Takashi, Kanagawa, 250-0193 (JP); TAKANO, Ikuo, Kanagawa, 250-0001 (JP)
(74) Representative: Klunker IP Patentanwälte PartG mbB

(57) **Abstract**

A method of producing a transdermal absorption sheet includes a preparatory step of forming the sheet portion in advance by drying and solidifying a base solution in a thin film state, a drug solution arrangement step of arranging a drug solution on a surface of a mold having needle-like recessed portions, a drug solution filling step of filling the needle-like recessed portions with the drug solution while pressingly expanding the drug solution on the surface of the mold by pressing the drug solution on the surface of the mold to the surface of the mold with the sheet portion, a drying step of drying an undried drug solution filling the needle-like recessed portions with the sheet portion to form needle-like protruding portions on a lower surface of the sheet portion, and a peeling-off step of peeling off the sheet portion and the needle-like protruding portions from the mold.

## Description

### BACKGROUND OF THE INVENTION

### 1. Field of the Invention

The present invention relates to a method of producing a transdermal absorption sheet and particularly to a technique for improving production efficiency in a method of producing a transdermal absorption sheet.

### 2. Description of the Related Art

In recent years, a transdermal absorption sheet in which a plurality of fine needle-like protruding portions (also referred to as microneedles) including a drug is arranged two-dimensionally on the surface of a sheet portion has attracted attention. A transdermal absorption sheet is used to deliver a drug in needle-like protruding portions into a skin by attaching a sheet portion to the skin to insert the needle-like protruding portions into the skin.

In a general method of producing a transdermal absorption sheet, a mold (molding form) in which needle-like recessed portions (also referred to as needle hole portions) having an inverted shape of needle-like protruding portions are formed is used. After the needle-like recessed portions of the mold are filled with a drug solution containing a drug, the drug solution is dried and solidified, a base solution for forming a sheet portion is applied, dried, and solidified, and then the sheet is peeled off from the mold to produce a transdermal absorption sheet.

Accordingly, the amount of drug solution filling the needle-like recessed portions is related to a variation in dosage of the drug in the transdermal absorption sheet. Therefore, particularly, in a step of filling the needle-like recessed portions of the mold with the drug solution, it is necessary to accurately fill each mold with a very trace amount of the drug solution corresponding to the dosage of drug in a fixed amount.

JP2015-217042A, JP2015-136422A, and JP2013-074924A are examples of methods of producing transdermal absorption sheets.

In JP2015-217042A, a drug solution filling step of filling needle-like recessed portions of a mold with a drug solution, a drug solution drying step of drying the filled drug solution to form a first layer, a base solution filling step of filling the needle-like recessed portions on the first layer with a base solution, a base solution drying step of drying the filled base solution to form a second layer on the first layer, and a peeling-off step are performed in this order, and each step from the drug solution filling step to the base solution drying step is performed in an environment of a temperature of 1°C or higher and 10°C or lower. In addition, in JP2015-217042A, in the drug solution filling step, the needle-like recessed portions are filled with the drug solution by moving the nozzle along the surface of the mold while supplying the drug solution to the surface of the mold from a slit-shaped nozzle, and thus filling efficiency is increased.

In JP2015-136422A, steps from a drug solution filling step to a peeling-off step are performed in the same manner as in JP2015-217042A but the weight-average molecular weight of the main component of the first layer is set to be smaller than the weight-average molecular weight of the main component of the second layer. In addition, in the JP2015-136422A, the drug solution is injected into each needle-like recessed portion by an ink jet method or using a dispenser.

In JP2013-074924A, steps from a drug solution filling step to a peeling-off step are performed in the same manner as in JP2015-217042A but in the drug solution filling step, after the drug solution is supplied to the surface of the mold, the needle-like recessed portions are filled with a predetermined amount of drug solution by moving a squeegee along the surface of the mold.

### SUMMARY OF THE INVENTION

However, in the methods of producing transdermal absorption sheets disclosed in JP2015-217042A, JP2015-136422A, and JP2013-074924A of the related art, the tact time (step operation time) in at least one of the drug solution filling step or the drying step of the drug solution and the base solution is long, which is a problem when enhancing the production efficiency of the transdermal absorption sheet.

That is, considering the drug solution filling step, as in the drug solution filling step disclosed in JP2013-074924A, the use of a squeegee for drug solution filling allows the tact time to be shortened, but a drug is also contained in the drug solution scraped off by the squeegee. Accordingly, each mold cannot be accurately filled with a very trace amount of a drug solution corresponding to the dosage of the drug of the produced transdermal absorption sheet in a fixed amount. Thus, a variation in dosage of the drug easily occurs in the produced transdermal absorption sheet. In addition, in the case of using a squeegee in JP2013-074924A, foreign substances are generated due to rubbing between the mold and the squeegee, and the generated foreign substances easily enter a transdermal absorption sheet to be produced.

On the other hand, as in JP2015-136422A, the method of injecting a drug solution into each needle-like recessed portion by an ink jet method or using a dispenser is not effective for the reason that each needle-like recessed portion of the mold can be accurately filled with a very trace amount of a drug solution corresponding to a dosage of a drug in a fixed amount but this process takes long time.

In addition, considering the drying step of the drug solution and the base solution, any countermeasure for shortening the tact time in any method of the production methods disclosed in JP2015-217042A, JP2015-136422A, and JP2013-074924A is not taken actually. For example, when drying is performed at a high temperature to shorten the tact time of the drug solution drying step, there is a concern of deterioration in the effect of the drug that is likely to be weak to heat.

The present invention is made in consideration of such circumstances and an object thereof is to provide a method of producing a transdermal absorption sheet capable of remarkably improving production efficiency compared to the related art since the tact time of a drug solution filling step and a drying step can be shortened without deteriorating filling accuracy and the effect of a drug.

In order to achieve the object, according to an aspect of the present invention, there is provided a method of producing a transdermal absorption sheet in which a plurality of fine needle-like protruding portions is arranged two-dimensionally on a surface of a sheet portion, the method comprising: a preparatory step of forming the sheet portion in advance by drying and solidifying a base solution that is a polymer solution in a thin film state; a drug solution arrangement step of arranging a drug solution that is a polymer solution including a drug on a surface of a mold on which a plurality of fine needle-like recessed portions having an inverted shape of the needle-like protruding portions is arranged two-dimensionally; a drug solution filling step of filling the needle-like recessed portions with the drug solution while pressingly expanding the drug solution on the surface of the mold by pressing the drug solution arranged on the surface of the mold to the surface of the mold with the sheet portion prepared in advance in the preparatory step; a drying step of drying an undried drug solution filling the needle-like recessed portions with the sheet portion with which the solution is pressed to the surface of the mold to form the needle-like protruding portions on a lower surface of the sheet portion; and a peeling-off step of peeling off the sheet portion and the needle-like protruding portions from the mold.

According to the transdermal absorption sheet of the aspect of the present invention, the tact time can be significantly shortened in the drying step at the time of production of the transdermal absorption sheet by forming the sheet portion dried and solidified in the preparatory step in advance. That is, the base solution of the sheet portion has a larger amount than the drug solution and is hardly dried. However, the base solution not containing a drug can be dried at a high temperature. Accordingly, the tact time can be shortened by drying at a high temperature by forming the sheet portion separately from the production of the transdermal absorption sheet. Thus, the drying time can be significantly shortened in the drying step without deteriorating the effect of the drug.

In addition, instead of filling each needle-like recessed portion with the drug solution B as in the related art, the needle-like recessed portions are filled with the drug solution by arranging the drug solution on the surface of the mold and pressingly expanding the arranged drug solution to the surface of the mold with the sheet portion prepared in advance. Thus, each mold can be accurately filled with a very trace amount of the drug solution in a fixed amount. Accordingly, the tact time of the drug solution filling step can be shortened without deteriorating filling accuracy in the drug solution filling step. In addition, since the drug solution spreads to each needle-like recessed portion by pressing and pressingly expanding the drug solution to the surface of the mold with the sheet portion, foreign substances are not generated without causing rubbing with the mold surface unlike the case of using as a squeegee which has been described in the related art.

It is possible to provide a method of producing a transdermal absorption sheet capable of remarkably improving production efficiency compared to the related art since the tact time of the drug solution filling step and the drying step can be shortened without deteriorating filling accuracy and the effect of the drug.

In the aspect of the present invention, it is preferable that in the drug solution arrangement step, a plurality of liquid droplets is arranged in a filling region in which the needle-like recessed portions are arranged in two-dimensionally on the surface of the mold. By arranging the plurality of liquid droplets as described above, when the drug solution is pressed and pressingly expanded on the surface of the mold with the sheet portion, the drug solution easily spreads to each needle-like recessed portion. In this case, it is preferable that the number of the plurality of liquid droplets is 4 to 9.

In the aspect of the present invention, it is preferable that in the drug solution filling step, a mold with a frame that surrounds a periphery of the mold is used as the mold. Thus, when the drug solution is pressed and pressingly expanded on the surface of the mold with the sheet portion, the drug solution can be prevented from leaking out from a space between the sheet portion and the mold surface.

In the aspect of the present invention, it is preferable that in the drug solution filling step, a mold in which a total volume of the plurality of needle-like recessed portions is larger than a volumetric amount of drug solution in an amount of drug solution measured is used as the mold. For example, the total volume of the plurality of needle-like recessed portions is set to be larger than the volumetric amount of the drug solution in the amount of drug solution measured by forming a plurality of needle-like recessed portions for leakage prevention the same that are as the needle-like recessed portions in a non-filling region in which the needle-like recessed portions are not arranged in two-dimensionally on the surface of the mold.

Here, the term "amount of drug solution measured" refers to an amount of drug solution corresponding to the total amount of drug solution filling the plurality of the needle-like recessed portions.

When the drug solution is pressed and pressingly expanded on the surface of the mold with the sheet portion, the drug solution expanded to the non-filling region flows into the needle-like recessed portions for leakage prevention and thus the drug solution can be prevented from leaking out from a space between the sheet portion and the mold surface. In this case, since the drug solution filling the needle-like recessed portions for leakage prevention is inserted into the skin in use of the transdermal absorption sheet, the effect of the transdermal absorption sheet does not change.

In the aspect of the present invention, it is preferable that in the drug solution filling step, the sheet portion is sucked and held on a suction plate of an elastic material in which a cross-sectional shape of a suction surface is a curved shape recessed in a direction in which a central portion separates from the surface of the mold, and when the liquid droplets of the drug solution are pressed to the surface of the mold with the sheet portion, a pressing force is applied to the surface of the mold from a peripheral edge portion of the sheet portion to a central portion. Thus, since the pressing force is moved from the peripheral edge portion of the mold surface to the central portion by the sheet portion, the drug solution can be prevented from leaking out from a space between the sheet portion and the mold surface.

In the aspect of the present invention, it is preferable that a thickness portion that is thicker than a center portion is formed in an outer edge portion of the sheet portion formed in advance. Thus, when the liquid droplets of the drug solution is pressed to the surface of the mold with the sheet portion sucked and held on a suction plate, the pressing force applied to the outer edge portion of the surface of the mold is larger than the pressing force applied to the center portion and thus the drug solution can be prevented from leaking out from a space between the sheet portion and the surface of the mold.

In the aspect of the present invention, it is preferable that in the drug solution filling step, a back surface of the mold is sucked. Thus, the tip ends of the needle-like recessed portions are easily filled with the drug solution and also the drug solution hardly leaks out from a space between the sheet portion and the mold surface when the drug solution is pressed and pressingly expanded on the surface of the mold with the sheet portion.

In the aspect of the present invention, it is preferable that in the preparatory step, a reinforcing material is embedded in the sheet portion. Thus, warping occurring by the sheet portion absorbing a solvent (for example, moisture) in the drug solution can be prevented from occurring.

According to the method of producing a transdermal absorption sheet of the present invention, it is possible to remarkably improve production efficiency compared to the related art since the tact time of the drug solution filling step and the drying step can be shortened without deteriorating filling accuracy and the effect of the drug.

### BRIEF DESCRIPTION OF THE DRAWINGS

Fig. 1 is a partially enlarged view of a transdermal absorption sheet having a needle-like protruding portion.
Fig. 2 is a cross-sectional view of the needle-like protruding portion shown in Fig. 1.
Fig. 3 is a perspective view of the transdermal absorption sheet having a needle-like protruding portion.
Fig. 4A is a cross-sectional view showing an original plate producing step in the production of a mold.
Fig. 4B is a cross-sectional view showing a transfer step in the production of a mold.
Fig. 4C is a cross-sectional view showing a mold produced in the production of the mold.
Fig. 5 is a top view of the mold when viewed from the surface thereof.
Fig. 6 is a cross-sectional view of a mold complex.
Fig. 7 is a configuration view illustrating the entire pressing device.
Fig. 8 is a flow chart of a method of producing a transdermal absorption sheet of the present invention.
Fig. 9A is a cross-sectional view in which a base solution is supplied to a mold frame in a preparatory step.
Fig. 9B is a cross-sectional view in which the base solution is dried in the preparatory step.
Fig. 9C is a cross-sectional view in which sheet portions formed in the preparatory step are laminated.
Fig. 9D is a cross-sectional view in which a reinforcing material is embedded in the sheet portion.
Fig. 9E is a perspective view showing an example of the reinforcing material.
Fig. 9F is a perspective view showing another example of the reinforcing material.
Fig. 10A is a cross-sectional view in which the mold complex is arranged on a suction table in a drug solution arrangement step.
Fig. 10B is a cross-sectional view in which the drug solution is arranged on the mold surface of the mold complex in the drug solution arrangement step.
Fig. 10C is a cross-sectional view in which the mold complex is set on a suction base of a pressing device in a drug solution filling step.
Fig. 10D is a cross-sectional view in which a liquid droplet of drug solution is pressed by the sheet portion held on a suction plate in the drug solution filling step.
Fig. 10E is a cross-sectional view in which the needle-like recessed portion is filled with the drug solution in the drug solution filling step.
Fig. 10F is a cross-sectional view in which the suction plate is separated from the mold complex in the drug solution filling step.
Fig. 10G is a cross-sectional view of a drying step.
Fig. 10H is a cross-sectional view of a peeling-off step.
Fig. 11A is a view in which a liquid droplet of drug solution is dripped on the mold surface.
Fig. 11B is a view in which five liquid droplets of a drug solution are dripped on the mold surface.
Fig. 11C is a view in which nine liquid droplets of a drug solution are dripped on the mold surface.
Fig. 12A is an illustration in which a countermeasure for drug solution leakage prevention is performed using a frame in the drug solution filling step.
Fig. 12B is an illustration showing a size relationship between the frame and the suction plate.
Fig. 13 is a top view in which a countermeasure for drug solution leakage prevention is performed using a needle-like recessed portion for leakage prevention formed on the mold surface in the drug solution filling step.
Fig. 14 is a cross-sectional view in which a countermeasure for drug solution leakage prevention is performed using a suction plate having a curved suction surface in the drug solution filling step.

### DESCRIPTION OF THE PREFERRED EMBODIMENTS

Hereinafter, a preferred embodiment of a method of producing a transdermal absorption sheet of the present invention will be described with reference to the accompanying drawings.

The present invention is described using the following preferred embodiment. The invention will be described with the following preferred embodiment. Modifications can be made by many methods without departing from the scope of the present invention, and embodiments other than the embodiment can be used. Accordingly, all of the modifications within the scope of the present invention are included in the claims.

In the drawings, components designated by the same reference numeral are similar components having similar functions. Furthermore, in the present specification, when a numerical range is described using "to", numerical values for an upper limit and a lower limit illustrated with "to" are also included in the numerical range.

First, an example of a transdermal absorption sheet produced by a method of producing a transdermal absorption sheet according to the embodiment will be described.

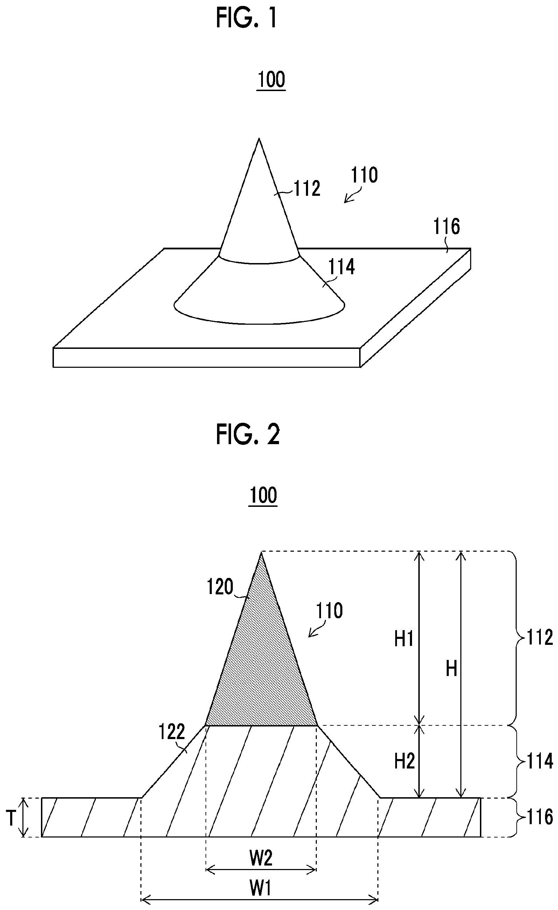

Fig. 1 is a partially enlarged view showing an example of a transdermal absorption sheet 100 and shows a needle-like protruding portion 110 (also referred to as a microneedle).

The transdermal absorption sheet 100 delivers a drug into the skin by being attached to the skin. As shown in Fig. 1, the transdermal absorption sheet 100 has a tapered-shaped needle portion 112, a frustum portion 114 connected to the needle portion 112, and a sheet-like sheet portion 116 connected to the frustum portion 114. The tapered-shaped needle portion 112 and the frustum portion 114 configure the needle-like protruding portion 110. The term "sheet-like" means a shape in which two facing principal surfaces having a large area (a first principal surface and a second principal surface) have a small thickness and are flat in overall shape and it is necessary that the principal surface is completely flat.

A plurality of frustum portions 114 is formed on the surface of the sheet portion 116 (only one frustum portion 114 is shown in Fig. 1). The frustum portion 114 has two bottom surfaces and has a stereoscopic structure surrounded by a pyramidal surface. Out of the two bottom surfaces of the frustum portion 114, a bottom surface (lower base) having a larger area is connected to the sheet portion 116. Out of the two bottom surfaces of the frustum portion 114, a bottom surface (upper base) having a smaller area is connected to the needle portion 112. That is, out of the two bottom surfaces of the frustum portion 114, a bottom surface in a direction in which the bottom surface is separated from the sheet portion 116 has a smaller area.

The needle portion 112 has a gradually tapered shape and the needle portion 112 has a shape having a large area at a bottom surface and having the smallest area at a tip end separated from the bottom surface. Since the bottom surface of the needle portion 112 having a large area is connected to the bottom surface of the frustum portion 114 having a small area, the needle portion 112 has a gradually tapered shape in a direction in which the needle portion is separated from the frustum portion 114. Accordingly, the needle portion 112 has a shape in which the needle-like protruding portion 110 formed by the needle portion and the frustum portion 114 is tapered from the sheet portion 116 to the tip end as a whole. 4 to 2,500 of a plurality of needle-like protruding portions 110 are provided on the sheet portion 116. However, the number of needle-like protruding portions is not limited to the above number.

In Fig. 1, the frustum portion 114 has a truncated cone shape, and the needle portion 112 has a cone shape. The shape of a tip end of the needle portion 112 can be appropriately changed to a curved surface having a radius of curvature of 0.01 µm to 50 µm, a flat surface, or the like in accordance with the degree of insertion of the needle portion 112 into the skin.

Fig. 2 is a cross-sectional view of the transdermal absorption sheet 100 shown in Fig. 1. In FIG. 2, for example, the transdermal absorption sheet 100 includes a first layer 120 containing a drug and a second layer 122 not containing a drug. The second layer 122 may contain a drug and only the first layer may include a needle portion.

The thickness T of the sheet portion 116 is preferably in a range of 10 µm to 2,000 µm and more preferably in a range of 10 µm to 1,000 µm. A width W1 of the bottom surface (lower base) in which the frustum portion 114 and the sheet portion 116 are in contact with each other is preferably in a range of 100 fly to 1,500 µm and more preferably in a range of 100 µm to 1,000 µm. A width W2 of the bottom surface (upper base) in which the frustum portion 114 and the needle portion 112 are in contact with each other is preferably in a range of 100 µm to 1,500 µm and more preferably in a range of 100 µm to 1,000 µm. It is preferable that the width W1 and the width W2 satisfy the relationship of W1 > W2 in the above numerical value range.

A height H of the needle-like protruding portion 110 is preferably in a range of 100 µm to 2,000 µm and more preferably in a range of 200 µm to 1,500 µm.

Although the transdermal absorption sheet 100 having the needle-like protruding portion 110 shown in Fig. 1 is shown in the embodiment, the shape of the needle-like protruding portion 110 of the transdermal absorption sheet 100 is not limited to this shape.

In addition, H1/H2 that is a ratio between a height H1 of the needle portion 112 and a height H2 of the frustum portion 114 is preferably in a range of 1 to 10 and more preferably in a range of 1.5 to 8. In addition, the height H2 of the frustum portion 114 is preferably in a range of 10 µm to 1,000 µm.

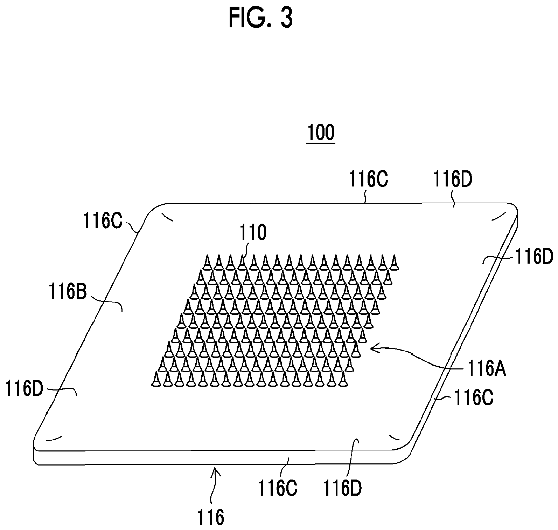

Fig. 3 is a perspective view of the entire transdermal absorption sheet 100. As shown in Fig. 3, the transdermal absorption sheet 100 includes the sheet portion 116 having the first principal surface and the second principal surface, and the plurality of needle-like protruding portions 110 arranged on the first principal surface of the sheet portion 116. The sheet portion 116 has an end portion 116C and includes a center portion 116A that is a region in which the plurality of needle-like protruding portions 110 is arranged two-dimensionally, and an outer edge portion 116B that is a region from the center portion 116A to the end portion 116C. The shape of the sheet portion 116 is defined by the end portion 116C in plan view. The sheet portion 116 of Fig. 3 has a rectangular shape in plan view but may have a polygonal shape, a circular shape, an elliptic shape, and the like. The shape of the sheet portion 116 is not limited as long as the center portion 116A in which the plurality of needle-like protruding portions 110 can be arranged and the outer edge portion 116B can be provided in the sheet portion. The transdermal absorption sheet 100 of the embodiment has a thickness portion 116D in the outer edge portion 116B. The thickness portion 116D is a portion in which the film thickness in the outer edge portion 116B of the sheet portion 116 is thick.

The needle-like protruding portion 110 is a portion protruding from the sheet portion 116 and the needle-like protruding portion 110 can be specified by defining a virtual auxiliary surface in contact with the first principal surface of the sheet portion 116.

Next, in the method of producing a transdermal absorption sheet of the present invention, preferred embodiments of the mold 13 used to perform the drug solution filling step, and a pressing device 10 for pressing a liquid droplet of drug solution arranged on the surface of the mold 13 to the surface of the mold with the sheet portion formed in advance will be described.

### Mold

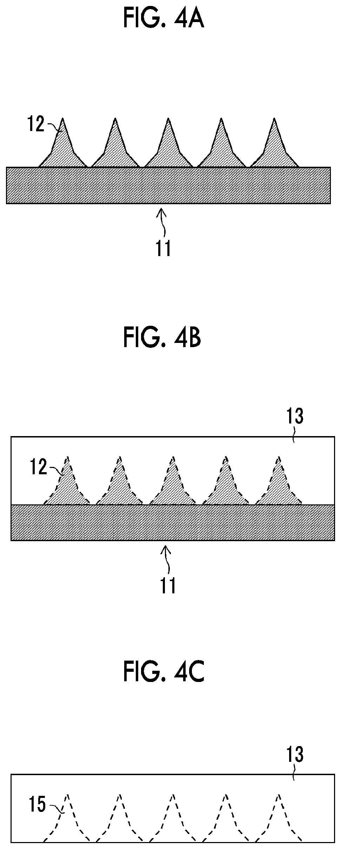

Figs. 4A to 4C are cross-sectional views showing a step of producing a mold (form) 13.

As shown in Fig. 4A, first, an original plate 11 for producing a mold 13 for producing a transdermal absorption sheet 100 is produced.

There are two kinds of methods of producing the original plate 11. The first method includes applying a photo resist to a Si substrate, and exposing and developing the photo resist. Then, etching by reactive ion etching (RIE) or the like is performed to produce a plurality of protruding portions 12, each having the same shape as the needle-like protruding portion 110 of the transdermal absorption sheet 100, in arrays on the surface of the original plate 11. In addition, when etching such as RIE is performed to form the protruding portion 12 corresponding to the needle-like protruding portion 110 of the transdermal absorption sheet on the surface of the original plate 11, the protruding portion 12 can be formed by performing etching from an oblique direction while rotating the Si substrate.

As the second method, there is a method including processing a metal substrate of stainless steel, an aluminum alloy, Ni, or the like using a cutting tool such as a diamond bit to produce a plurality of protruding portions 12 in arrays on the surface of the original plate 11.

Next, as shown in Fig. 4B, a mold 13 is produced using the original plate 11. In order to produce a normal mold 13, a method using Ni electroforming or the like is used. Since the original plate 11 has the protruding portions 12 having a conical shape with a sharp tip end, the shape is accurately transferred to the mold 13, and the mold 13 can be peeled off from the original plate 11. Four methods are considered for production at a low cost.

The first method is a method in which a silicone resin obtained by adding a curing agent to polydimethylsiloxane (PDMS, for example, SYLGARD 184, manufactured by Dow Corning Corporation) is poured into the original plate 11 and cured by a heating treatment at 100°C, and then the mold 13 is peeled off from the original plate 11. The second method is a method in which an ultraviolet curable resin that is curable by ultraviolet irradiation is poured into the original plate 11 and irradiated with ultraviolet light in a nitrogen atmosphere, and then the mold 13 is peeled off from the original plate 11. The third method is a method in which a material obtained by dissolving a plastic resin such as polystyrene or polymethylmethacrylate (PMMA) in an organic solvent is poured into the original plate 11 which has been coated with a release agent, and is dried to volatilize the organic solvent for curing, and then the mold 13 is peeled off from the original plate 11. The fourth method is a method in which an inverted article is made by Ni electroforming. In addition, in any of the three methods, the mold 13 can be easily produced any number of times.

In this manner, the mold 13 in which the needle-like recessed portions 15 having an inverted shape of the protruding portions 12 of the original plate 11 are arranged two-dimensionally is produced. The mold 13 produced in this manner is shown in Fig. 4C. Since the shape of the protruding portions 12 of the original plate 11 is the same as the shape of the needle-like protruding portions 110 of the transdermal absorption sheet, as shown in Fig. 4C, the mold 13 having the plurality of needle-like recessed portions 15 corresponding to the inverted shape of the needle-like protruding portions 110 of the transdermal absorption sheet 100 is produced.

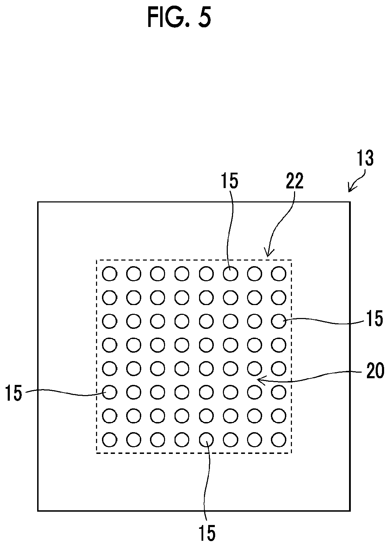

Fig. 5 is an overall view of the produced mold 13 when viewed from the surface thereof, and the plurality of fine needle-like recessed portions 15 having an inverted shape of the protruding portions 12 of the original plate 11 is arranged two-dimensionally. Here, in the surface of the mold 13, a region in which the needle-like recessed portions 15 are arranged two-dimensionally is referred to as a filling region 20 (inner side of the dotted line) and a region of the mold peripheral edge in which the needle-like recessed portions 15 are not arranged two-dimensionally is referred to as a non-filling region 22 (outer side of the dotted line).

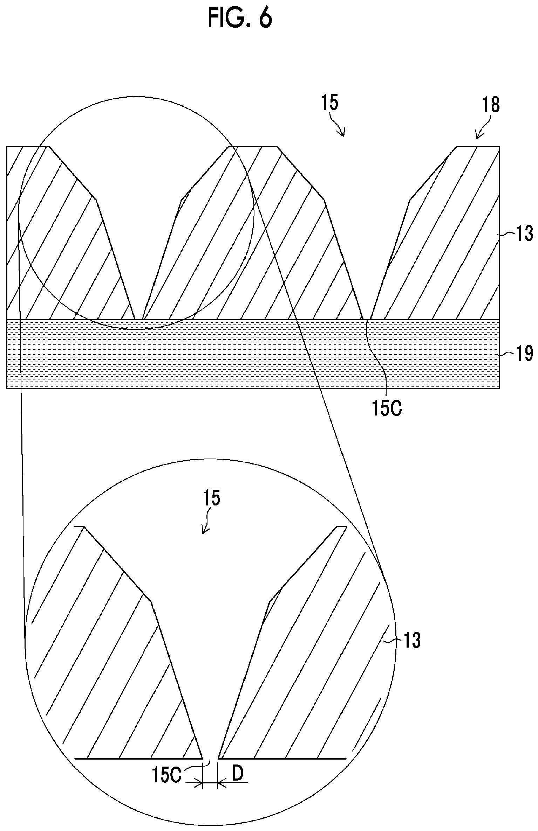

Fig. 6 shows a more preferred embodiment of a mold complex 18 in performing a method of producing a transdermal absorption sheet 100. As shown in Fig. 6, the mold complex 18 includes a mold 13 in which a through-hole 15C is formed at the tip end of the needle-like recessed portion 15 and a gas permeable sheet 19 that is bonded to the side of the through-hole 15C of the mold 13 and is made of a material that is gas permeable, but is not liquid permeable. Through the through-hole 15C, the tip end of the needle-like recessed portion 15 communicates with the atmosphere through the gas permeable sheet 19. The expression "tip end of the needle-like recessed portion 15" means a side that is tapered in a depth direction of the mold 13 and is opposite to a side from which a drug solution that is a polymer solution containing a drug is poured.

Using such a mold complex 18, only the air present in the needle-like recessed portion 15 can be removed from the needle-like recessed portion 15 via the through-hole 15C without permeation of the drug solution filling in the needle-like recessed portion 15. Transferability when the shape of the needle-like recessed portion 15 is transferred to a polymer material of the drug solution is improved, and thus it is possible to form a sharper needle-like protruding portion 110.

A diameter D of the through-hole 15C is preferably in a range of 1 to 50 µm. By adjusting the diameter within this range, air bleeding is easily performed, and the tip end portion of the needle-like protruding portion 110 of the transdermal absorption sheet 100 can be formed into a sharp shape. As the gas permeable sheet 19 made of a material that is gas permeable, but is not liquid permeable, for example, POREFLON (registered trademark, manufactured by Sumitomo Electric Industries, Ltd.) can be suitably used.

In addition, as described above, the through-hole 15C is formed in the mold 13 to improve filling properties and peelability of a drug solution B, and in the present invention, regardless of the presence of the through-hole 15C of the mold 13, the mold can be applied in the drug solution filling step.

As the material used for the mold 13, an elastic raw material and a metallic raw material can be used. Of these, an elastic raw material is preferable and a raw material with high gas permeability is more preferable.

The oxygen permeability, which is representative of the gas permeability, is preferably more than 1 × 10⁻¹² mL/s·m·Pa and more preferably more than 1 × 10⁻¹⁰ mL/s·m·Pa. When the mold 13 is made of a material high gas permeability, the drug solution can be sucked by suction from a back surface of the mold 13, and thus it is possible to promote the filling of the needle-like recessed portion 15 with the drug solution. In addition, the air present in the needle-like recessed portion 15 of the mold 13 can be removed from the mold 13. It is possible to produce a transdermal absorption sheet 100 with few defects.

Specific examples of such a material include materials obtained by melting general engineering plastics such as a silicone resin (for example, SYLGARD 184 (registered trademark), manufactured by Dow Corning Toray Co., Ltd. or 1310ST (item number), manufactured by Shin-Etsu Chemical Co., Ltd.), an ultraviolet curable resin, a polystyrene resin, polymethylmethacrylate (PMMA), an epoxy resin, a polyethylene terephthalate (PET) resin, a polyoxymethylene (POM) resin, TEFLON (registered trademark) resin (polytetrafluoroethylene), a polyethylene (PE) resin, a phenol resin, and a urethane resin, and materials obtained by dissolving any of the above resins in a solvent.

Among these, a silicone rubber-based raw material can be suitably used because of the durability thereof to transfers by repeated pressurization and the good peelability thereof from the raw material.

Examples of the metallic raw material include Ni, Cu, Cr, Mo, W, Ir, Tr, Fe, Co, MgO, Ti, Zr, Hf, V, Nb, Ta, α-aluminum oxide, zirconium oxide, stainless steel (STAVAX material), and alloys thereof.

### Drug Solution and Base Solution

The drug solution B is a polymer solution containing a drug.

The drug contained in the polymer solution is not particularly limited as long the drug is a substance having bioactivity. The drug is preferably selected from the group consisting of peptide, protein, nucleic acid, polysaccharide, a vaccine, a medical compound, and a cosmetic component. In addition, it is preferable that the medical compound belongs to a water-soluble low molecular weight compound. Here, the low molecular weight compound is a compound having a molecular weight of several hundreds to several thousands.

As the water-soluble polymer substance for forming a polymer solution, one that does not interact with the drug contained in the layer is preferably used. For example, in the case of using protein as the drug, when a chargeable polymer substance is mixed with the protein, the protein, and the polymer substance electrostatically interact with each other to form an aggregate, which is cohered and precipitated. Therefore, in the case in which a chargeable substance is used in the drug, a water-soluble polymer substance with no charge such as hydroxyethyl starch or dextran is preferably used.

A base solution A is a polymer solution not containing a drug and as the water-soluble polymer substance for forming the polymer solution, a water-soluble polymer substance such as chondroitin sulfate, hydroxyethyl starch, or dextran is preferably used.

### Pressing Device

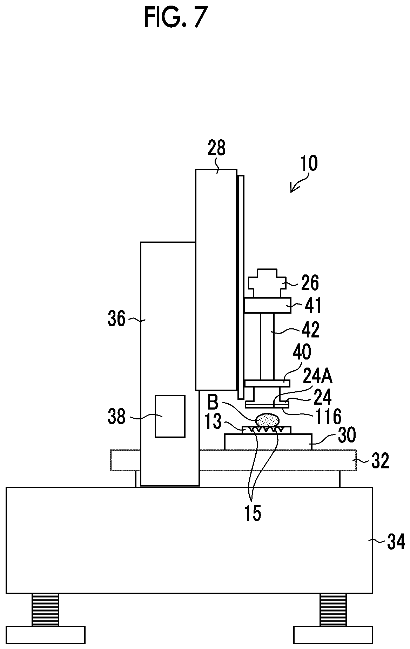

Fig. 7 is a configuration view showing an example of the entire configuration of a pressing device 10 used in the drug solution filling step.

As shown in Fig. 7, the pressing device 10 includes a suction plate 24, a vacuum pump 26 that imparts a suction force to the suction plate 24, a Z-axis driving unit 28 that vertically moves the suction plate 24 and the vacuum pump 26 in a vertical direction (Z-axis direction), a suction base 30 for mounting and fixing the mold 13 thereon, a load cell 32 that measures a pressing force for pressing the suction plate 24 to the surface of the mold 13, a stand 34 that supports the device, a support block 36 that is erected on the stand 34 to support the Z-axis driving unit 28, and a control unit 38 that is provided in the support block 36 to control the entire pressing device 10.

The suction plate 24 is arranged to be parallel with the surface of the mold 13 in which a suction surface 24A is fixed to the suction base 30 and is supported by the Z-axis driving unit 28 through a bracket 40. Thus, when the suction plate 24 is lowered by the Z-axis driving unit 28 and pressed to the surface of the mold 13, a uniform pressing force is applied to the entire surface of the mold 13.

The vacuum pump 26 is mounted on a bracket 41 provided above the suction plate 24 and the bracket 41 is supported by the Z-axis driving unit 28. The vacuum pump 26 and the suction plate 24 are connected to each other through a suction pipe 42. Thus, when the vacuum pump 26 vertically moves with the suction plate 24 and the vacuum pump 26 is driven, a suction force is generated on the suction surface 24A of the suction plate 24. The vacuum pump 26 is not limited to being supported by the Z-axis driving unit 28 and may be arranged above the stand 34 by being connected to the suction plate 24 through a flexible pipe (not shown).

The load cell 32 means a measuring tool that measures a pressing force applied to the mold 13 in the thickness direction. The pressing force to the mold 13 is an arbitrary pressure within a range of 1 to 1,000 kPa and is preferably controlled to be constant.

Next, the method of producing a transdermal absorption sheet of the embodiment will be described.

### Method of Producing Transdermal Absorption Sheet

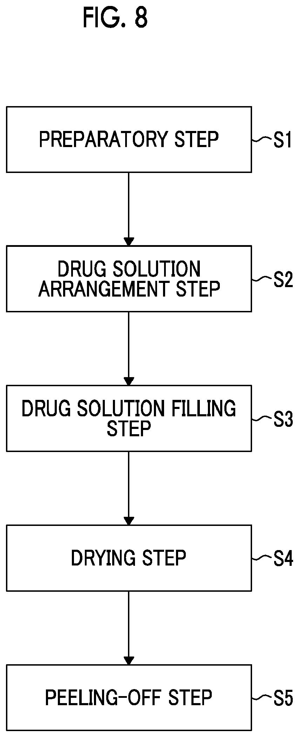

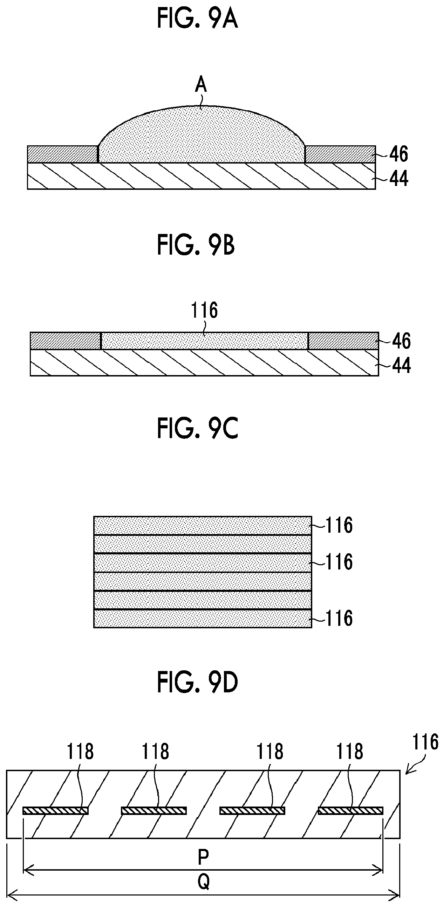

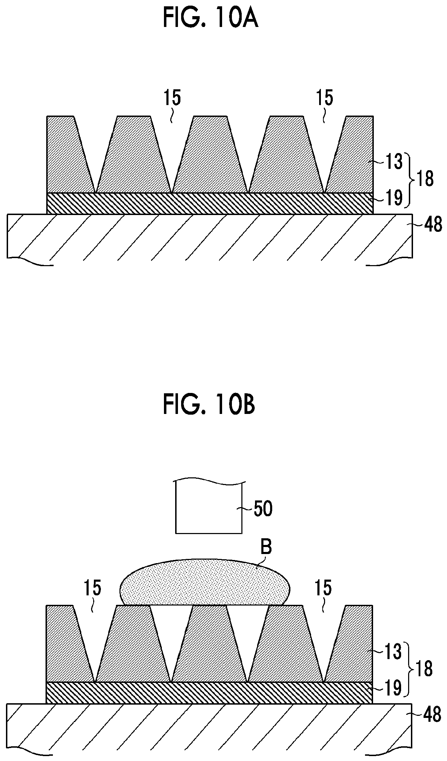

As shown in Fig. 8, in the method of producing a transdermal absorption sheet of the embodiment, at least five steps of a preparatory step (S1), a drug solution arrangement step (S2), a drug solution filling step (S3), a drying step (S4), and a peeling-off step (S5) are respectively performed in this order. Each step will be described using Figs. 9A to 9C and Figs. 10A to 10H. In Figs. 10A to 10H, the case of using the mold complex 18 as the mold 13 is shown but a normal mold 13 may be used.

### Preparatory step

The preparatory step S1 is a step of separately forming the sheet portion 116 out of the sheet portion 116 and the needle-like protruding portion 110 configuring the transdermal absorption sheet 100 in advance. That is, as shown in Fig. 9A, a mold frame 46 having the shape of the sheet portion 116 is placed on a base 44 having a flat surface and the base solution A that is a polymer solution not containing a drug is supplied to the mold frame 46. Then, as shown in Fig. 9B, the base solution A is dried and solidified in a thin film state along the shape of the mold frame 46 to form the sheet portion 116. Fig. 9C is a view in which the sheet portion 116 formed by drying and solidifying the solution is taken out from the mold frame 46 and a plurality of sheet portions 116 is laminated. The number of sheet portions 116 formed in advance is preferably equal to or more than the number of transdermal absorption sheets 100 to be produced.

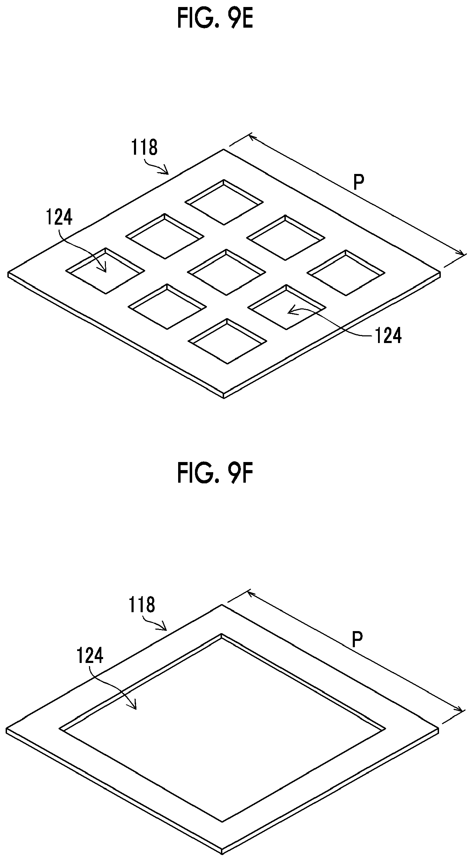

In the preparatory step S1, it is preferable that a reinforcing material 118 is embedded in the sheet portion 116. Fig. 9D is a cross-sectional view in which the plate-like reinforcing material 118 shown in Fig. 9E is embedded in the center portion of the sheet portion 116 in the thickness direction in the plane direction. Here, the plane direction is a direction parallel with the front and back surfaces of the sheet portion 116, but the plane direction is not necessarily completely parallel with the surfaces and may be parallel with the surfaces at a glance.

As shown in Fig. 9E, the reinforcing material 118 is formed to have a rectangular shape so as to match with the shape of the sheet portion 116 and vertical and horizontal sizes P of the reinforcing material 118 are formed to be slightly smaller than vertical and horizontal sizes Q of the sheet portion 116. Thus, the reinforcing material 118 is embedded not to be exposed from the sheet portion 116.

In addition, it is preferable that a through-hole 124 is formed in the reinforcing material 118. The reinforcing material 118 in Fig. 9E has a plurality of through-holes 124 (nine through-holes) in some cases and the reinforcing material 118 in Fig. 9F is formed into a rectangular frame shape with one through-hole formed therein.

As the method of embedding the reinforcing material 118 in the sheet portion 116, for example, only a half of the total amount of the base solution A is supplied to the mold frame 46 on the base 44 shown in Fig. 9A. Then, the supplied base solution A is dried until the solution loses its fluidity to form an underlayer of the sheet portion 116. The reinforcing material 118 is placed on the underlayer of the sheet portion 116 and the remaining base solution A is supplied to form an upper layer of the sheet portion 116. At the time of forming the upper layer, the base solution A is poured into the through-hole 124 of the reinforcing material 118 to bond the upper layer and the underlayer. In this state, the upper layer and the underlayer are completely dried and a sheet portion 116 including the reinforcing material 118 is produced. When the sheet portion 116 is produced as described above, the upper layer and the underlayer of the sheet portion 116 are bonded to each other through the through-hole 124 to fix the reinforcing material 118. Thus, the reinforcing material 118 is not easily peeled off from the sheet portion 116.

### Drug Solution Arrangement Step

The drug solution arrangement step S2 is a step of arranging the drug solution B that is a polymer solution containing a drug on the surface of the mold 13 on which the plurality of fine needle-like recessed portions 15 is arranged in two-dimensionally. In the embodiment, an example in which the drug solution B is arranged on the surface of the mold 13 by dripping liquid droplets of the drug solution B in the amount of drug solution measured corresponding to the total amount of drug solution filling the plurality of needle-like recessed portions 15 is described. That is, as shown in Fig. 10A, the mold complex 18 is arranged on a dripping table 48. Then, as shown in Fig. 10B, the liquid droplets of the drug solution B in the amount of drug solution measured is arranged on the surface of the mold 13 from a dripping nozzle 50 that drips the drug solution B. In this case, a plurality of liquid droplets is preferably arranged on the surface of the mold 13 and 4 to 9 liquid droplets are more preferably arranged.

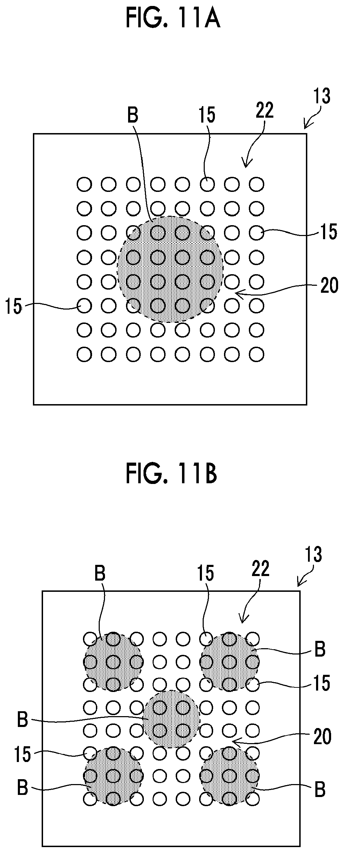

Figs. 11A to 11C shows the number of liquid droplets of the drug solution B arranged on the surface of the mold 13 and the places to be arranged.

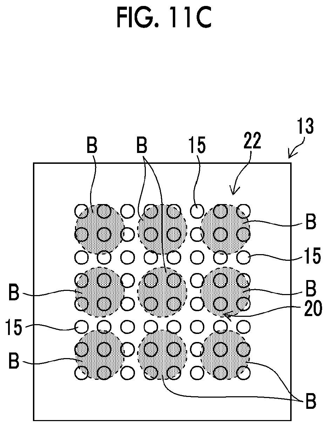

Fig. 11A shows the case in which one liquid droplet of drug solution B in the amount of drug solution measured is arranged in the filling region 20 in which the needle-like recessed portions 15 are arranged in two-dimensionally. Fig. 11B shows the case in which the liquid droplets of the drug solution B in the amount of drug solution measured are arranged such that a total of five liquid droplets of the drug solution B are arranged at the center portion and four corners of the filling region 20. Fig. 11C shows the case in which the liquid droplets of the drug solution B in the amount of drug solution measured are arranged such that a total of nine liquid droplets of three liquid droplets each in vertical and horizontal direction are arranged in the filling region 20.

As shown in Fig. 11B or 11C, if a plurality of liquid droplets of the drug solution B is arranged in the filling region 20 in which the needle-like recessed portions 15 are arranged in two-dimensionally, the liquid droplets of the drug solution B are easily evenly expanded in the filling region 20 when the liquid droplets of the drug solution B dripped on the surface of the mold 13 are pressed to the surface of the mold 13 with the sheet portion 116 in the following drug solution filling step S3.

### Drug Solution Filling Step

The drug solution filling step S3 is a step of filling the needle-like recessed portions 15 while pressingly expanding the liquid droplets on the surface of the mold 13 by pressing the liquid droplets arranged on the surface of the mold 13 to the surface of the mold 13 on which the liquid droplets of the drug solution B are arranged with the sheet portion 116 prepared in advance in the preparatory step.

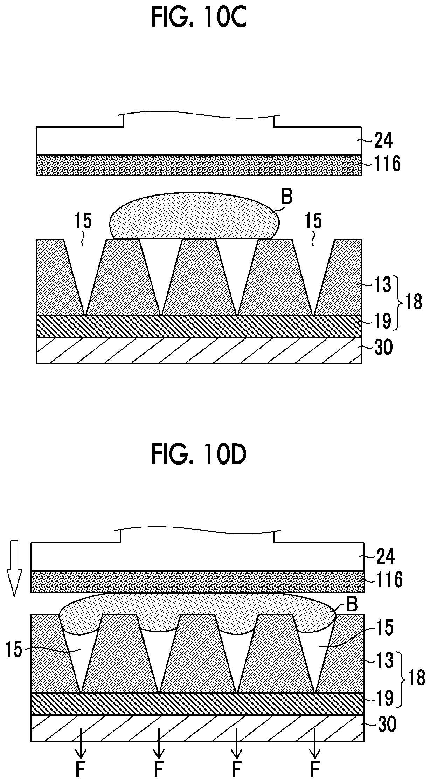

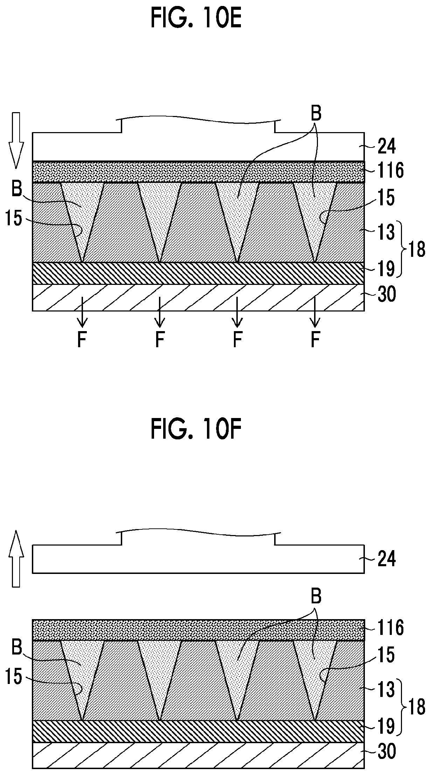

That is, as shown in Fig. 10C, the mold complex 18 in which the liquid droplets of the drug solution B are arranged on the surface of the mold 13 is arranged right under the suction plate 24 of the suction base 30 of the pressing device 10 to fix the mold using a suction force. On the other hand, the sheet portion 116 prepared in advance in the preparatory step is sucked and held on the suction surface 24A of the suction plate 24. Next, the suction plate 24 is lowered by the Z-axis driving unit 28 and approaches the surface of the mold 13. As shown in Fig. 10D, the liquid droplets of the drug solution B arranged on the surface of the mold 13 are pressed to the surface of the mold 13 with the sheet portion 116 sucked and held on the suction plate 24. Thus, the liquid droplets of the drug solution B moves, spread into all the needle-like recessed portions 15 while being pressingly expanded on the surface of the mold 13, and flows into the needle-like recessed portions 15. Further, the suction plate 24 is lowered by Z-axis driving unit 28 until the pressing force applied to the surface of the mold 13 by the suction plate 24 reaches a desired pressing force. Whether or not a desired pressing force is applied is confirmed by the load cell 32. Thus, as shown in Fig. 10E, the needle-like recessed portions 15 are filled with all the liquid droplets of the drug solution B arranged on the surface of the mold 13.

In the drug solution filling step S3, when the sheet portion 116 absorbs the solvent (for example, moisture) in the drug solution B during pressing the liquid droplets of the drug solution B to the surface of the mold 13 with the sheet portion 116, there is a concern of occurrence of warping.

However, as described in the preparatory step S1, warping can be prevented from occurring in the sheet portion 116 by embedding the reinforcing material 118 in the sheet portion 116. Accordingly, the pressing force pressing the surface of the mold 13 is uniform on the sheet portion 116 through the drug solution B and thus all the needle-like recessed portions 15 can be uniformly filled with the drug solution B.

In addition, in the drug solution filling step, as shown in Figs. 10D and 10E, the liquid droplets of the drug solution B pressingly expanded on the surface of the mold 13 easily flow into the tip ends of the needle-like recessed portions 15 by removing air in the needle-like recessed portions 15 by suction through the gas permeable sheet 19 by applying a suction force F to the suction base 30.

When the drug solution filling step S3 ends, as shown in Fig. 10F, the suction force of the suction plate 24 is released and the suction plate 24 is lifted by the Z-axis driving unit 28. Accordingly, the sheet portion 116 is arranged to be in close contact with on the surface of the mold 13 in which the plurality of needle-like recessed portions 15 is filled with the drug solution B.

As described above, the liquid droplets of the drug solution B are pressed and pressingly expanded on the surface of the mold by the sheet portion 116 held on the suction plate 24 to spread to all the needle-like recessed portions 15. Accordingly, when a pitch (interval) between the needle-like recessed portions 15 is excessively wide, the liquid droplets of the drug solution B hardly spread to all the needle-like recessed portions 15 in some cases. In addition, when the pitch (interval) between the needle-like recessed portions 15 is excessively wide, the filling region 20 on the surface of the mold becomes wide and the non-filling region becomes narrow. Thus, the drug solution B easily leaks out from a space between the sheet portion 116 and the surface of the mold 13 to the outside in some cases. Accordingly, the pitch between the needle-like recessed portions 15 to be formed on the mold 13 is preferably smaller than the pitch between needle-like recessed portions of a mold used for producing a transdermal absorption sheet of the related art. Specifically, the pitch between the needle-like recessed portions 15 is preferably 1 mm or less.

In addition, in order to make the drug solution B hardly leak out from a space between the sheet portion 116 and the surface of the mold 13 to the outside, the amount of drug solution arranged (that is, the amount of drug solution measured) is preferably an amount in which the drug solution does not overflow from the needle-like recessed portions 15. Therefore, the volume of the drug solution in the amount of drug solution measured is preferably equal to or less than the total volume of the plurality of needle-like recessed portions 15. Specifically, it is preferable that the amount of drug solution B dripped is set such that the dried and solidified drug solution B is accommodated in a range from an area approximately 0.2 mm close to the tip ends (bottom side) of the needle-like recessed portions 15 from the surface of the mold 13 to the tip ends of the needle-like recessed portions 15.

In addition, since the drug solution B does not leaks out from a space between the sheet portion 116 and the surface of the mold 13 to the outside, it is preferable to adopt the following embodiment.

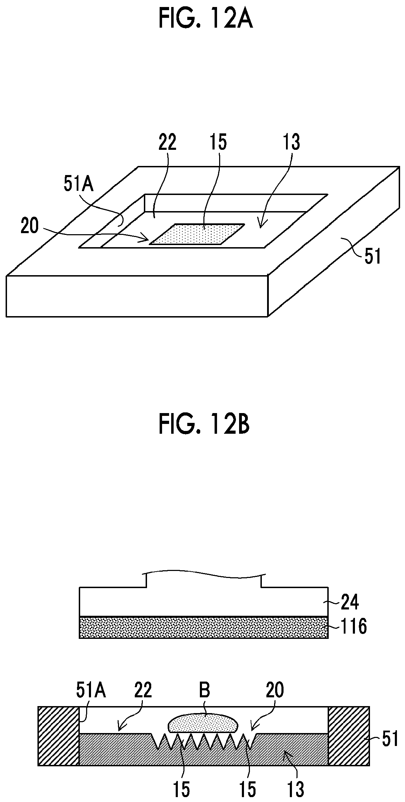

In Fig. 12A, a mold 13 with a frame 51 that surrounds the periphery of the mold is used. In this case, as shown in Fig. 12B, it is necessary that the sheet portion 116 and the suction plate 24 are made slightly smaller than an inner wall surface 51 A of the frame 51 in size and shape so that the sheet portion 116 and the surface of the mold 13 are in close contact with each other when the liquid droplets of the drug solution B dripped on the surface of the mold 13 are pressed with the sheet portion 116 sucked and held on the suction plate 24.

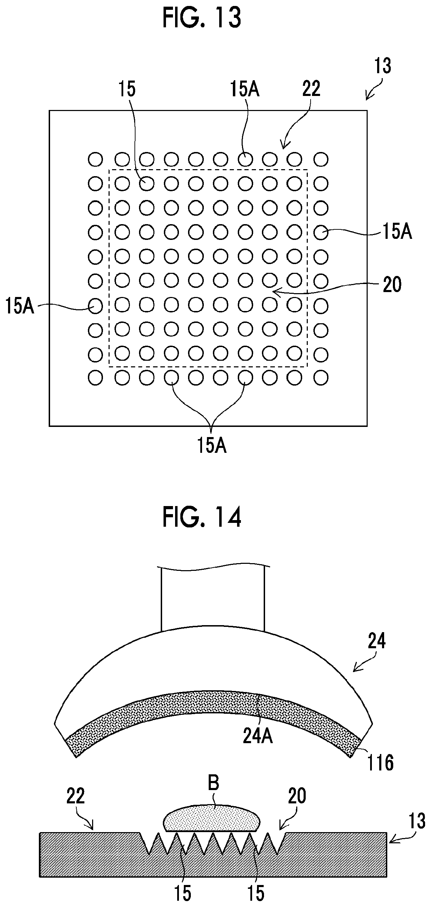

Fig. 13 shows an example of an embodiment in which in the drug solution filling step, as the mold 13, a mold 13 in which the total volume of the plurality of needle-like recessed portions 15 is larger than the volumetric amount of the drug solution B in the amount of drug solution measured is used. That is, as shown in Fig. 13, in the non-filling region 22 in which the needle-like recessed portions 15 are not arranged two-dimensionally on the surface of the mold 13, a plurality of needle-like recessed portions 15A for leakage prevention the same as the needle-like recessed portions 15 are formed. Thus, during pressing and pressingly expanding the liquid droplets of the drug solution B on the surface of the mold 13 by the sheet portion 116, the drug solution B is pressingly expanded in the non-filling region 22 outside the filling region 20 having the needle-like recessed portions 15 and flows into the needle-like recessed portions 15A for leakage prevention. Accordingly, it is possible to prevent the drug solution B from leaking out from the space between the sheet portion 116 and the surface of the mold 13. In this case, since the drug solution B filling the needle-like recessed portions 15A for leakage prevention is also inserted into the skin in use of the transdermal absorption sheet 100, the effect of the transdermal absorption sheet 100 does not change. In Fig. 13, one round of needle-like recessed portions 15A for leakage prevention is formed so as to surround the filling region 20. However, two or more rounds of needle-like recessed portions may be formed.

In Fig. 14, in the drug solution filling step S3, as the suction plate 24, a suction plate 24 of an elastic material (for example, rubber) whose a cross-sectional shape of the suction surface 24A is a curved shape recessed in a direction in which the center portion separates from the surface of the mold 13 is used. Accordingly, when the liquid droplets of the drug solution B are pressed to the surface of the mold 13 with the sheet portion 116 sucked and held on the suction plate 24, the pressing force moves from the peripheral edge portion of the surface of the mold to the center portion and thus the drug solution B can be prevented from leaking out from the space between the sheet portion 116 and the surface of the mold 13.

In addition, it is preferable that a thickness portion 116D that is thicker than a center portion 116A is formed in an outer edge portion 116B of the sheet portion 116 formed in advance (refer to Fig. 3) to prevent solution leakage. Thus, when the liquid droplets of the drug solution B are pressed to the surface of the mold 13 with the sheet portion 116 sucked and held on the suction plate 24, the pressing force applied to the outer edge portion (non-filling region 22) of the surface of the mold 13 is larger than the pressing force applied to the center portion (filling region 20), and thus the drug solution B can be prevented from leaking out from the space between the sheet portion 116 and the surface of the mold 13.

Further, since the drug solution is sucked by suction from the back surface of the mold 13 using the mold complex 18, the pressing force when the liquid droplets of the drug solution B are pressed to the surface of the mold 13 with the sheet portion 116 sucked and held on the suction plate 24 is applied to the inside of the needle-like recessed portions 15 and thus the drug solution B can be prevented from leaking out from the space between the sheet portion 116 and the surface of the mold 13.

### Drying Step

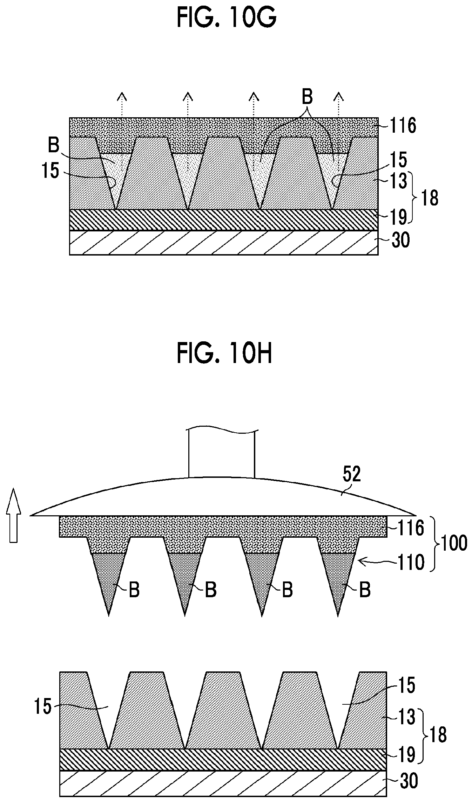

The drying step S4 is a step of drying and solidifying an undried drug solution B filling the needle-like recessed portions 15 with the dried and solidified sheet portion 116 with which the liquid droplets of the drug solution B are pressed to the surface of the mold 13 to form needle-like protruding portions 110 on the lower surface of the sheet portion 116. That is, as indicated by the dotted arrow in Fig. 10G, drying is performed such that a solvent (typically, water) of the drug solution B permeates the solidified sheet portion 116 while evaporating from the surface of the sheet portion 116. In the process of the drying, in a state in which a part of the lower surface side (mold surface side) of the solidified sheet portion 116 is dissolved by the solvent of the drug solution B and some of the dissolved base solution A and the undried drug solution B are mixed, the drug solution B is dried and solidified. Since the volume of the drug solution B filling the needle-like recessed portions 15 is reduced by drying and solidifying the drug solution B, as shown in Fig. 10G, the dissolved base solution A flows into the needle-like recessed portions 15 and forms root portions of needle-like protruding portions 110 of a transdermal absorption sheet to be produced. Thus, needle-like protruding portions 110 in which the drug solution B is solidified are formed on the lower surface of the sheet portion 116 to be integral with the sheet portion 116.

As described above, drying is performed such that the solvent (typically, water) of the drug solution B permeates the solidified sheet portion 116 while evaporating from the surface of the sheet portion 116. Accordingly, it is necessary that the sheet portion 116 has a sufficient thickness (volumetric amount) not to cause the entire sheet portion 116 to become a liquid state even when the sheet portion 116 absorbs the solvent of the drug solution B due to permeation of the solvent. If the entire sheet portion 116 becomes a liquid state by absorbing the solvent of the drug solution B, there is a concern that the drug in the drug solution B spreads to the entire sheet portion 116. Accordingly, it is preferable that only the surface layer portion of the lower surface of the sheet portion 116 becomes a liquid state.

### Peeling-Off Step

The peeling-off step S5 is a step of peeling off the sheet portion 116 and the needle-like protruding portions 110 after being subjected to the drying step S4 from the mold complex 18. As shown in Fig. 10H, the back surface of a polymer sheet of a transdermal absorption sheet 100 in which the sheet portion 116 and the needle-like protruding portions 110 are integrally formed is sucked and held on a suction disk 52 and the suction disk 52 is moved in a direction in which the suction disk separates from the mold 13. Thus, the mold 13 is peeled off from the polymer sheet to produce a transdermal absorption sheet 100.

In Fig. 10G showing the drying step S4 and Fig. 10H showing the peeling-off step, the suction base 30 that sucks and holds the mold complex 18 is shown but the suction base 30 may not be provided.

According to the method of producing a transdermal absorption sheet described above, the drying step for drying the drug solution can be performed once at the time of production of the transdermal absorption sheet by forming the sheet portion 116 dried and solidified in the preparatory step in advance. In addition, at drying of the drug solution B and drying of base solution A, the base solution A whose amount is larger than that of the drug solution requires a long drying time. Further, since the drug solution B contains a drug, the drying time cannot be shortened by increasing the drying temperature. However, since the base solution A does not contain a drug, by the drying temperature can be increased by forming the sheet portion separately from the production of the transdermal absorption sheet, thereby shortening the drying time.

Accordingly, by forming the sheet portion 116 dried and solidified in the preparatory step (S1) in advance, and performing each step of the drug solution arrangement step (S2), the drug solution filling step (S3), the drying step (S4), and the peeling-off step (S5) in this order, the drying time at the time of production of the transdermal absorption sheet can be significantly shortened in the drying step S4 without deteriorating the effect of the drug.

In addition, instead of filling each needle-like recessed portion 15 with the drug solution B as in the related art, the liquid droplets of the drug solution B in the amount of drug solution measured are dripped on the surface of the mold 13 and the dripped liquid droplets are pressed to the surface of the mold 13 with the sheet portion 116 prepared in advance. Thus, the liquid droplets move and flow into the needle-like recessed portions 15 while pressingly expanding the liquid droplets on the surface of the mold 13, and thus the needle-like recessed portions 15 are filled with the drug solution B. In addition, by dripping the drug solution B in the amount of drug solution measured corresponding to the total amount of drug filling the plurality of needle-like recessed portions 15, a required filling amount of drug solution to exhibit the medicinal effect of the produced transdermal absorption sheet can be exactly acquired. Thus, a variation in dosage of the drug of the transdermal absorption sheet 100 to be produced does not occur.

Accordingly, the tact time of the drug solution filling step S3 can be shortened without deteriorating filling accuracy in the drug solution filling step S3.

Thus, in the method of producing a transdermal absorption sheet of the present invention, since the tact time of the drug solution filling step S3 and the drying step S4 can be shortened without deteriorating filling accuracy and the effect of the drug, production efficiency can be remarkably improved compared to the related art.

In the drug solution filling step (S3), since the drug solution B spreads to each needle-like recessed portion 15 by pressingly expanding the liquid droplets on the surface of the mold 13 by pressing the liquid droplets of the drug solution B to the surface of the mold 13 with the sheet portion 116, foreign substance are not generated without causing rubbing with the mold surface unlike the case of using as a squeegee which has been described in the related art.

### Explanation of References

- 10:: pressing device
- 11:: original plate
- 12:: protruding portion
- 13:: mold
- 15, 15A:: needle-like recessed portion
- 15C:: through-hole
- 18:: mold complex
- 19:: gas permeable sheet
- 20:: filling region
- 22:: non-filling region
- 24:: suction plate
- 24A:: suction surface
- 26:: vacuum pump
- 28:: Z-axis driving unit
- 30:: suction base
- 32:: load cell
- 34:: stand
- 36:: support block
- 38:: control unit
- 40, 41:: bracket
- 42:: suction pipe
- 44:: base
- 46:: mold frame
- 48:: dripping table
- 50:: dripping nozzle
- 51:: frame
- 51A:: inner wall surface
- 52:: suction disk
- 100:: transdermal absorption sheet
- 110:: needle-like protruding portion
- 112:: needle portion
- 114:: frustum portion
- 116:: sheet portion
- 116A:: center portion
- 116B:: outer edge portion
- 116C:: end portion
- 116D:: thickness portion
- 118:: reinforcing material
- 120:: first layer
- 122:: second layer
- 124:: through-hole
- A:: base solution
- B:: drug solution
- S1:: preparatory step
- S2:: drug solution arrangement step
- S3:: drug solution filling step
- S4:: drying step
- S5:: peeling-off step

## Claims

1. A method of producing a transdermal absorption sheet in which a plurality of fine needle-like protruding portions is arranged two-dimensionally on a surface of a sheet portion, the method comprising:
a preparatory step of forming the sheet portion in advance by drying and solidifying a base solution that is a polymer solution in a thin film state;
a drug solution arrangement step of arranging a drug solution that is a polymer solution including a drug on a surface of a mold on which a plurality of fine needle-like recessed portions having an inverted shape of the needle-like protruding portions is arranged two-dimensionally;
a drug solution filling step of filling the needle-like recessed portions with the drug solution while pressingly expanding the drug solution on the surface of the mold by pressing the drug solution arranged on the surface of the mold to the surface of the mold with the sheet portion prepared in advance in the preparatory step;
a drying step of drying an undried drug solution filling the needle-like recessed portions with the sheet portion with which the solution is pressed to the surface of the mold to form the needle-like protruding portions on a lower surface of the sheet portion; and
a peeling-off step of peeling off the sheet portion and the needle-like protruding portions from the mold.

2. The method of producing a transdermal absorption sheet according to claim 1,
wherein in the drug solution arrangement step, a plurality of liquid droplets is dripped in a filling region in which the needle-like recessed portions are arranged in two-dimensionally on the surface of the mold.

3. The method of producing a transdermal absorption sheet according to claim 2,
wherein the number of the plurality of liquid droplets is 4 to 9.

4. The method of producing a transdermal absorption sheet according to any one of claims 1 to 3,
wherein in the drug solution filling step, a mold with a frame that surrounds a periphery of the mold is used as the mold.

5. The method of producing a transdermal absorption sheet according to any one of claims 1 to 3,
wherein in the drug solution filling step, a mold in which a total volume of the plurality of needle-like recessed portions is larger than a volumetric amount of drug solution in an amount of drug solution measured is used as the mold.

6. The method of producing a transdermal absorption sheet according to claim 5,
wherein the total volume of the plurality of needle-like recessed portions is set to be larger than the volumetric amount of drug solution in the amount of drug solution measured by forming a plurality of needle-like recessed portions for leakage prevention that are the same as the needle-like recessed portions in a non-filling region in which the needle-like recessed portions are not arranged in two-dimensionally on the surface of the mold.

7. The method of producing a transdermal absorption sheet according to any one of claims 1 to 3,
wherein in the drug solution filling step, the sheet portion is sucked and held on a suction plate of an elastic material in which a cross-sectional shape of a suction surface is a curved shape recessed in a direction in which a center portion separates from the surface of the mold, and when the liquid droplets of the drug solution are pressed to the surface of the mold with the sheet portion, a pressing force is applied to the surface of the mold from a peripheral edge portion of the sheet portion to a center portion.

8. The method of producing a transdermal absorption sheet according to any one of claims 1 to 3,
wherein a thickness portion that is thicker than a center portion is formed in an outer edge portion of the sheet portion formed in advance.

9. The method of producing a transdermal absorption sheet according to any one of claims 1 to 8,
wherein in the drug solution filling step, a back surface of the mold is sucked.

10. The method of producing a transdermal absorption sheet according to any one of claims 1 to 9,
wherein in the preparatory step, a reinforcing material is embedded in the sheet portion.
